# EUROPEAN PATENT SPECIFICATION

(11) **EP 3 768 859 B1**
(45) Date of publication and mention of the grant of the patent: **01.03.2023**
(21) Application number: 19714758.0
(22) Date of filing: 22.03.2019
(51) Int. Cl.: C12Q 1/6853

(54) **METHODS FOR AMPLIFICATION OF NUCLEIC ACIDS WITH ENDONUCLEASE-MEDIATED SHIFTING EQUILIBRIUM (EM-SEQ)**
VERFAHREN ZUR AMPLIFIKATION VON NUKLEINSÄUREN MIT ENDONUKLEASE-VERMITTELTEM VERLAGERTEM GLEICHGEWICHT (EM-SEQ)
MÉTHODES D'AMPLIFICATION D'ACIDES NUCLÉIQUES AVEC ÉQUILIBRE DE DÉCALAGE À MÉDIATION PAR ENDONUCLÉASE (EM-SEQ)

(30) Priority: 22.03.2018 GB 201804585
(43) Date of publication of application: 27.01.2021
(73) Proprietor: DNAe Diagnostics Limited, London W12 7RZ (GB)
(72) Inventor: LIPINSKI, Kamil Andrzej, London W12 7RZ (GB)
(74) Representative: Stratagem IPM Limited
(86) International application number: PCT/GB2019/050822
(87) International publication number: WO 2019/180455

(56) References cited:
- EP-A1- 0 684 315
- WO-A2-2004/094456
- BHUSHAN J. TOLEY ET AL: "Isothermal strand displacement amplification (iSDA): a rapid and sensitive method of nucleic acid amplification for point-of-care diagnosis", THE ANALYST, vol. 140, no. 22, 1 January 2015 (2015-01-01), pages 7540-7549, XP055382231, UK ISSN: 0003-2654, DOI: 10.1039/C5AN01632K

## Description

### FIELD OF THE INVENTION

The present invention relates to the design of molecular biology assays based on isothermal amplification of nucleic acids with Strand Displacement Amplification (SDA) and its various embodiments and method improvements. The invention describes a novel method for SDA termed Endonuclease-Mediated Shifting Equilibrium Amplification (EM-SEq), which improves exponential kinetics and specificity of the reaction and enables amplification on solid surfaces.

### BACKGROUND TO THE INVENTION

### Strand Displacement Amplification (SDA)

SDA is a fast and efficient isothermal nucleic acid amplification method that uses a sequence-specific DNA endonuclease, such as a restriction or nicking enzyme, in combination with a DNA polymerase lacking 5'-3' exonuclease activity and able to displace (rather than degrade) a complementary DNA strand encountered downstream while extending a free 3'-end over a template strand. SDA has been originally described to operate at the temperature of 37-40 degrees Celsius due to temperature profile of enzymes available at that time, causing high levels off-target amplification. However, a thermophilic SDA (tSDA) has been described that uses thermostable enzymes and allows for the reaction to be carried out at 50 degrees Celsius and above.

### Mechanism of SDA

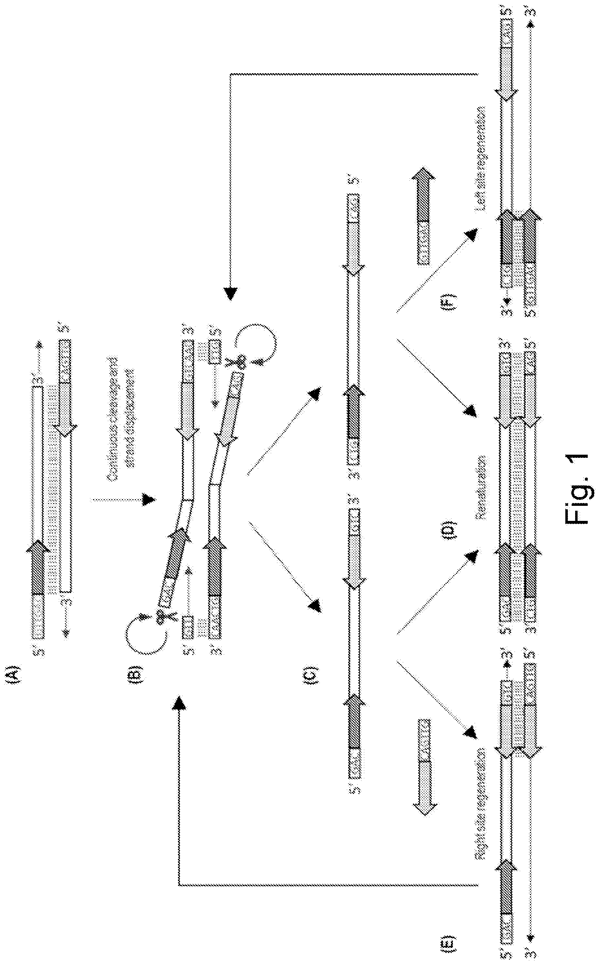

When amplifying target sequences lacking endonuclease recognition sites, such as many sequences found in nature, the reaction typically begins by an initial temperature denaturation step that allows for two single-stranded priming oligonucleotides (later referred to as primers) to specifically bind to two ends of a target DNA sequence. At their 5'-ends the two primers carry a short (typically between 6 and 8 nucleotides long) recognition sequence serving as a binding site for a given DNA endonuclease used in the reaction. Upon primer extension by DNA polymerase, a species of double-stranded DNA is created that contains the target DNA sequence flanked on both ends by chosen DNA endonuclease recognition sequence. This molecule then serves as a substrate for subsequent SDA reaction (Figure 1A).

The amplification cycle of SDA proceeds with the DNA endonuclease binding to its recognition sites and cleaving a phosphodiester bond preferentially in only one backbone strand of the DNA duplex (strand with proximal 5'-end). Generation of such single-stranded cuts (later referred to as "nicks") instead of cleaving DNA on both strands may be achieved in at least two ways. First, a non-palindromic restriction enzyme may be used that shows sensitivity to certain modified nucleotides. For instance, in its first documented application SDA has been demonstrated with a restriction enzyme Hindi, which has a non-palindromic recognition sequence GTT*GAC, where asterisk denotes cleavage site. In order to avoid cleaving the complementary strand carrying GTCAAG sequence, deoxyadenosine 5'-[α - thio]triphosphate (dATPαS) is used instead of standard adenosine 5'-triphosphate (dATP), both in primer synthesis and amplification reaction. As a result, Hincll restriction enzyme is unable to cut the thioester bond formed between the C and the first A in the complementary GTCAAG sequence, while retaining the ability to introduce a nick between second T and G in the target GTTGAC sequence on the first strand.

As an alternative to using modified nucleotides, restriction enzymes naturally found or artificially engineered to cleave only one DNA strand (also known as nicking enzymes) may be used instead of standard restriction enzymes. Such SDA embodiment is also known as nicking enzyme amplification reaction (NEAR) or nicking endonuclease dependent amplification (NDA). A number of suitable nicking enzymes have been described, including Nt.BspQl, Nt.BbvCl, Nb.BbvCl, Nt.BstNBI and many more. Nicking one strand of the DNA duplex leaves a hydroxyl group on a newly formed free 3'-end, which is then extended during SDA by the aforementioned DNA polymerase with a strand-displacing activity, such as Bst polymerase. As a result, the strand downstream of the nick is released into solution. As the new complementary strand is synthesized, the endonuclease recognition site is regenerated, allowing for cycles of nick formation and extension that linearly produce single complementary strands downstream of the introduced nick. Since the target sequence is flanked by endonuclease recognition sites on both ends, reaction produces both strands simultaneously from two types of "amplifying units" (Figure 1B).

However, DNA strands produced in that process are flanked by a truncated (cleaved) endonuclease recognition site and therefore cannot further amplify (Figure 1C). To achieve exponential amplification, the recognition site needs to be regenerated. Most commonly this is achieved due to presence of the two primers, which bind to the product strands with their complementary 3'-ends (Figure 1E, F). Extending the product strand from its 3'-end when the primer is bound regenerates one of the cleaved endonuclease recognition sites, turning a cleaved product into an "amplifying unit".

Importantly, since both of the linearly produced DNA strands with cleaved recognition sites are perfectly complementary, rather than binding to free primers they can also readily anneal with each other (renaturate), leading to a "dead-end" double-stranded product molecule that cannot amplify (Figure 1D). High primer concentrations are required to outcompete product renaturation. Moreover, in contrast to Polymerase Chain Reaction (PCR), reaction occurs isothermally and the system cannot be re-set by heat denaturation at each cycle to re-attempt primer binding. As a result, SDA produces at least three different types of product molecules, with the dead-end product often dominating in the late stages of the reaction when product-primer ratio becomes high. Such dead-end product also lacks any sequences or chemical moieties present on 5' primer ends. This puts significant limitations on SDA output, for example when attempting amplification on surfaces with the use of immobilized primers.

A typical nicking and extension reaction can be seen in for example US2009/0017453. In such conditions, the amplicons generated are short (all examples around 25 base pairs in length) and the majority of amplicons do not contain the initial amplification adaptors, which are required for subsequent analysis, thus the method can perform amplification such that the presence of amplified material can be detected, the method can not be used to prepare amplicons for further sequencing.

A version of SDA termed true isothermal SDA (iSDA) has been described that allows to omit initial heat denaturation step with two primer pairs, one of which acts as "flaps", in Analyst (2015) vol 140 no 22, pages 7540-7549. The method requires the breathing of the sample initially in order for a pre-exponential primer to insert into the genomic sequence and extend. This avoids the need to heat denature in order to hybridise the initial extension primers. Once the initial pre-exponential product is formed and displaced by extension of a bumper primer to displace the extended strand without using the restriction site, this double stranded product is amplified by standard SDA. Thus the problems of SDA as described are not overcome as the majority of the amplified material can not be further sequenced due to the lack of adapted ends for further amplification.

In contrast to SDA, which uses a sequence-specific DNA endonuclease, such as a restriction or nicking enzyme, in combination with a strand displacing DNA polymerase lacking 5'-3' exonuclease activity, other isothermal amplification techniques have been reported. Examples include US2007/0054301, which describes a method where the templates have breathable ends such that new primers can strand invade and therefore extend. This is in contrast to SDA, which uses a sequence-specific DNA endonuclease to nick the primers to give a free 3' end for extension. The requirement to strand invade a whole primer and extend, rather than nick and extend an already hybridised primer makes the method different to, and less efficient than SDA.

Described herein is a method for thermophilic SDA termed Endonuclease-Mediated Shifting Equilibrium Amplification (EM-SEq) that by means of primer design limits generation of the dead-end product, thereby improving the exponential kinetics of the reaction, reducing the number of product types being generated and shifting the balance between products to those containing 5'-terminal primer sequences.

### SUMMARY

Described is an improved method for isothermal amplification of nucleic acids. The method shifts the balance of products from double stranded products having dead ends which are fully complementary to products having the correctly adapted ends suitable for further use. The method relies on incorporating breathable terminal regions which are sufficiently transiently single stranded at the isothermal amplification temperature to hybridise to a primer and undergo a snap extension. Such low melting, breathable terminal regions can be viewed as frayed ends, and are introduced to the strands before amplification commences. The extension can then proceed in the same way as other means of strand displacement amplification, via repeated steps of nicking and extension where the extension uses a strand displacing polymerase. The low melting ends introduced on the templates however prevent the accumulation of dead end amplicons.

Described is a method for the strand displacement amplification of a population of double stranded nucleic acid sequences comprising:
a. modifying the ends of the strands in the population such that at least one of the ends contains a low melting point region of sequence which, at a temperature of above 37 °C and below 80 °C, is at least transiently single stranded;
b. copying the population of nucleic acid molecules having low melting point ends using one or more amplification primers which hybridise to the low melting point ends, wherein the primers have a 5' single stranded section beyond the 3' end of the template population of nucleic acid molecules such that the 3' end of the template is extended to form a complete recognition site for an endonuclease, and the 3' end of the primer is extended by strand displacement to copy the template;
c. using the complete recognition site for the endonuclease to nick the extended strand, thereby releasing a free 3'-OH group within the primer; and
d. extending the freed 3'-OH group by strand displacement to re-copy the template,
wherein steps b, c and d are performed isothermally, thereby resulting in the strand displacement amplification of the population of double stranded nucleic acid sequences.

The method can be performed using a single amplification primer, thereby copying one strand of the population of double stranded nucleic acid sequences. Alternatively the method can be performed using two amplification primers, thereby copying both strands of the population of double stranded nucleic acid sequences. The amplification primers can be immobilised on a solid support.

The molecules termed amplification primers can be blocked at their 3' end by a suitable blocking moiety, thereby preventing 3' end extension of the primer before the primer is nicked by the endonuclease to shorten the blocked primer and thereby enable amplification.

Any method of strand displacement can be used. For example extension steps b and d can be performed using a strand displacing polymerase. The polymerase can be Bst polymerase or Klenow fragment polymerase. In order for amplification of double stranded sample to occur, the sample must be double stranded apart from the low melting ends, which are sufficiently single stranded for primer hybridisation to occur at the isothermal amplification temperature.

The ends of the strands can be modified using any suitable method. For example, the ends of the strands in the population can be modified by adaptor ligation. Alternatively the modified ends of the strands in the population can be obtained using extension of a primer having said modification.

The low melting point regions can be prepared using suitable nucleic acid sequences. The desired difference in melting temperature of the two region types may be achieved in a number of ways. For example, (i) through significantly different GC% content, (ii) use of modified bases that alter thermal stability of the DNA duplex, such as but not limited to locked nucleic acids (LNA), unlocked nucleic acids (UNA) or 8-aza-7-deazaguanosine, and (iii) partial complementarity between LL and LR regions present in the primer molecules and those in the template DNA. For example the low melting point region may contain a mis-matched base pair such that the sequences are not fully complementary. For example the low melting point ends may have less than 30% GC content. For example the ends may be 20 nucleotides-long DNA sequences having less than 30% GC content.

The method relies on a sufficient amount of the low melting point terminal region being single stranded that hybridisation of a primer can occur at the amplification temperature. The amplification temperature is generally above 37 °C and below 80 °C. The temperature at which the ends are at least transiently single stranded can be the same as the amplification temperature. The low melting point region can be single stranded at the isothermal amplification temperature. The temperature can be 37-65 °C. The isothermal amplification temperature can be 50-65 °C. The low melting point region can be a sequence which does not occur in nature.

In order to facilitate efficient hybridization of the primer molecule to the transiently single-stranded low melting point region of the template, the complementary primer sequence can be composed of nucleotides with modified bases that stabilise duplex formation. The primer has a 5'- overhang. Once the primer has transiently hybridised, a snap extension occurs which extends the 3' end of the template, thereby increasing the stability of the hybridised primer by increasing the length of the hybridised region.

The extended primer produces the full sequence of a double stranded restriction endonuclease site. Part of the sequence of the double stranded restriction endonuclease site can be in the modified low melting ends. Alternatively the full sequence can be in the primer. As an example if a six base double stranded recognition site is chosen, three bases can be from the modified low melting point ends of the population, and three bases can be in the single stranded primer. Strand extension from the 3'-end of the low melting region extends the template, the first three bases making the complete recognition site. Alternatively all six bases can be in the single stranded primer. Strand extension from the 3'-end of the low melting region extends the template, the first six bases making the complete recognition site.

It is beneficial if the full double stranded recognition site is not attached to the ends of the template molecules, otherwise strand extension can occur without the step of extending the 3'- end of the template to make the overhanging primer double stranded. The method relies on the full double stranded recognition site being made by extension of the 3'-end of the template. Extension of the template opposite the primer produces a region of sequence that remains double stranded throughout the isothermal amplification.

The nick in the extended strand can be generated using an enzyme. The nick in the extended strand can be generated using a nicking endonuclease. The nicking endonuclease can be selected from Nt.BspQI, Nt.CviPII, Nt.BstNBI, Nb.BsrDl, Nb.Btsl, Nt.Alwl, Nb.BbvCl, Nt.BbvCI or Nb.Bsml.

The nick can be generated where one strand is capable of being cut and one strand is resistant to being cut. Cut resistant strands can be made when the extension is performed using a non-natural dNTP which generates a non cleavable nucleic acid backbone. Such a non-natural dNTP includes an 5'-[α -thio]triphosphate, giving a cut resistant thioester bond.

The endonuclease recognition sequence can be
3'- CAGTTG-5'
5'-GTCAAC-3'

The endonuclease recognition sequence can be
3'- CAG*TTG-5'
5'-GTCsAAC-3'
where * is the cut site and s is phosphothioate.

Where the endonuclease recognition sequence is as shown above, the sequence at the end of the modified 5' strand can be 5'-GAC. The sequence at the 3' end of the modified strand can be 3'-CTG.

Each primer in such a scheme requires an internal sequence 3'-CAGTTG-5'. The sequence at the end of the modified 5' strand can be before the 3'-CAGTTG-5' sequence such that this sequence is fully single stranded until copied by template extension. Alternatively the primer can hybridise at an point within the sequence as long as the 3'-CAGTTG-5' is not fully double stranded.

The amplified material can be further analysed, for example by sequencing. Thus in the methods disclosed, the immobilised extension products can be subsequently sequenced. Where the amplification is performed using two immobilised primers, both strands are copied. Thus both strands can be sequenced. A pair of reads can be generated, a first sequencing read from a first strand, and a second read from the other strand, thereby generating a pair of reads, one read from each strand of the population of double stranded nucleic acid sequences.

Also disclosed are kits for performing the method. Disclosed is a kit for the modification of nucleic acid sequences comprising:
a. a first pair of nucleic acid adaptor molecules for modifying the ends of a a population of double stranded nucleic acid sequences, wherein the 5' end of a first strand of the adaptor pair contains part of a recognition site for an endonuclease, the central part of the adaptor pair contains a low melting point region of sequence which, at a temperature of above 37 °C and below 80 °C, is at least transiently single stranded, and the 3' end of the first strand of the adaptor pair and the 5' end of the second strand of the adaptor pair can undergo ligation to both strands of the population of double stranded nucleic acid sequences; and
b. a ligase enzyme.

The kit may include additional components, for example one or more amplification primers or one or more enzymes. The kits may include a strand displacing polymerase. One or more of the reagents may be immobilised, for example the amplification primers can be immobilised.

### FIGURES

Figure 1: Modus operandi of standard exponential Strand Displacement Amplification (SDA). Vertical lines denote hydrogen bonds between paired bases. (A) In its most basic form the SDA reaction begins with an initial denaturation step, which allows for binding of template DNA to two primer molecules that contain an endonuclease recognition site at their 5- ends, such as a binding site for a non-palindromic restriction or nicking enzyme (Hindi restriction enzyme site GTT*GAC, where asterisk denotes the cleavage site, is shown as an example). Strand extension by DNA polymerase incorporates the recognition site into the complementary strand of the target DNA duplex. (B) The reaction is designed such that the endonuclease can cut only one of the DNA strands. This can be achieved through e.g. use of a non-palindromic restriction enzyme and modified nucleotides, or a nicking enzyme. The endonuclease cleaves one strand of the DNA duplex. Where the same restriction site is present at both ends, both strands are nicked at opposing ends. The nicks have free 3'- ends that can be extended by a strand displacing polymerase such as Bst. (C). Continuous cycles of cleavage and extension produce in a linear fashion two complementary strands that contain the target sequence, both of the primer sequences and truncated (cleaved) endonuclease recognition sites on both ends. (D) These single-stranded products may readily renaturate to form a double stranded product that can no longer amplify. (E,F) Alternatively, if the product binds a complementary primer instead, one of the cleaved endonuclease recognition sites can be regenerated. Recognition site regeneration in product molecules is necessary to achieve exponential amplification. Because product renaturation (shown in D) is a dead end, reaction kinetics is sub-exponential and the reaction produces at least three different types of double stranded product molecules and single stranded products.

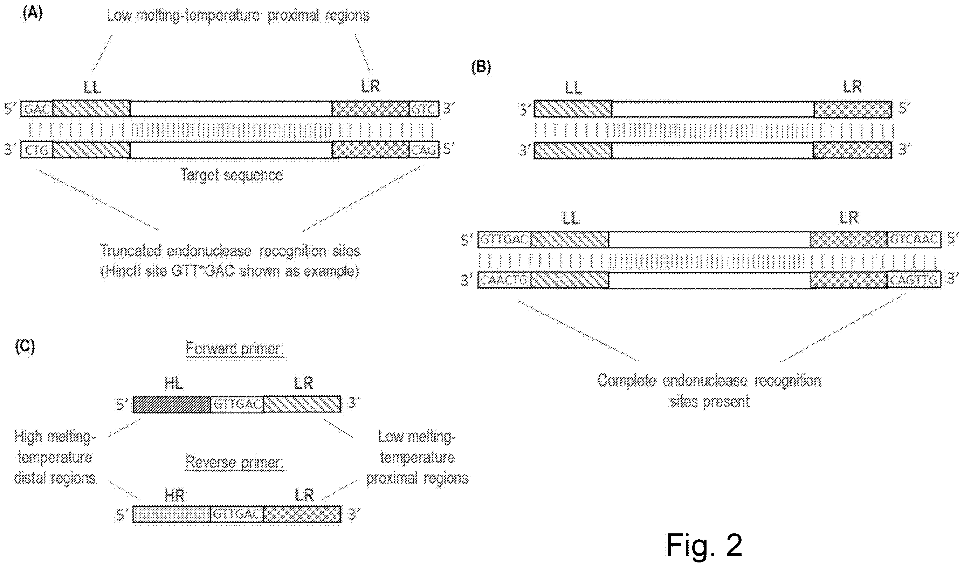

Figure 2: Design of template DNA and primers for Endonuclease-Mediated Shifting Equilibrium Amplification (EM-SEq). Vertical lines denote hydrogen bonds between paired bases. (A) Template DNA serving as a substrate in EM-SEq amplification. White boxes: target sequence; Diagonal boxes: Low-Tm Left (LL) regions; Checker board boxes; Low Tm Right (LR) regions. Hincll restriction enzyme site GTT*GAC where asterix denotes cleavage site, is shown as an example. (B) In some embodiments there may be no part of the recognition site initially present in the DNA (top) and its incorporation may occur as as EM-SEq progresses. In other embodiments, a partial or complete recognition site may already be present in the template DNA (bottom). Figure shows template DNA as being double stranded. However in some embodiments single stranded template DNA may be used. (C) Primers used in EM-SEq reaction. Solid dark gray boxes: High-Tm Left (HL) regions; solid light gray boxes: High-TM Right (HR) regions.
Figure 3: The principle of shifting equilibrium kinetics in EM-SEq. (A) Low-melting temperature regions LL and LR flanking the target sequence allow for transient opening of the ends of the DNA duplex at both ends of the template DNA (DNA breathing). (B) Dynamic binding equilibrium between template DNA molecules and amplification primers. (C) Kinetics of EM-SEq according to Le Chatelier's principle. A: self-bound state, B: primer bound state, C: snap-extended product
Figure 4: Amplification cycle of EM-SEq. (A) Similar to standard SDA, continuous cleavage and strand displacement generates two complementary single stranded DNA molecules, which in EM-SEq contain target sequence flanked by low-melting regions LL and LR as well as truncated (cleaved) endonuclease recognition sites on both ends. (B) In contrast to standard SDA, renaturated products are converted by snap-extension to products with regenerated endonuclease recognition sites. This shifts the balance between products being generated towards molecules that can be amplified exponentially.
Figure 4b shows extension using primers generated by cleavage using an endonuclease.
Figure 5: Completion of EM-SEq generated amplicons with a thermostable DNA polymerase. Heat inactivation of both the strand-displacing polymerase and the restriction enzyme simultaneously activates the hot-start thermostable polymerase, which subsequently fills the DNA nicks (asterisks) through nick translation. Active enzymes shown in bold.

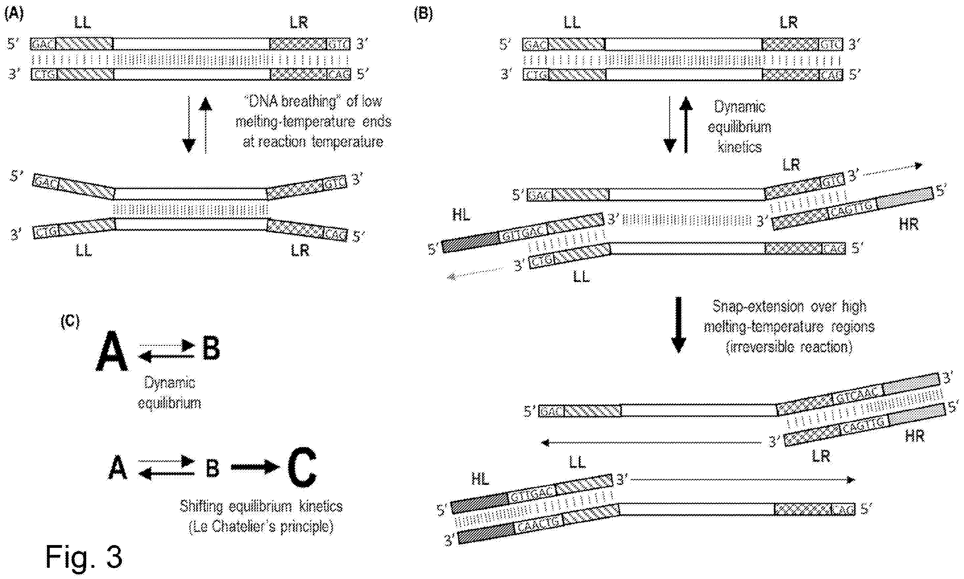

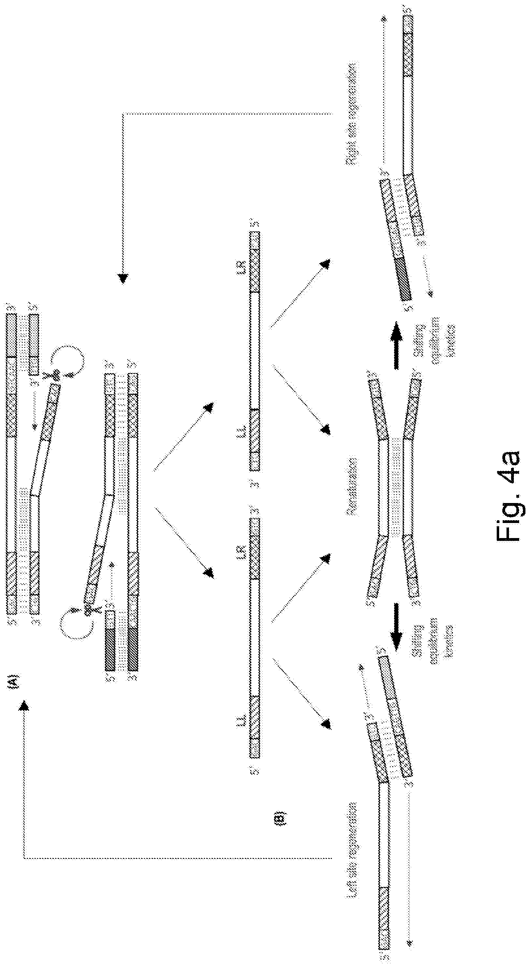

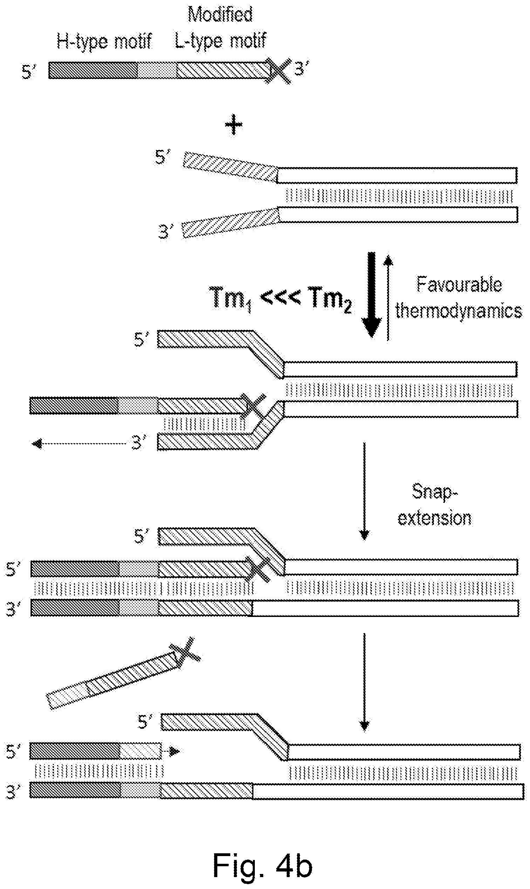

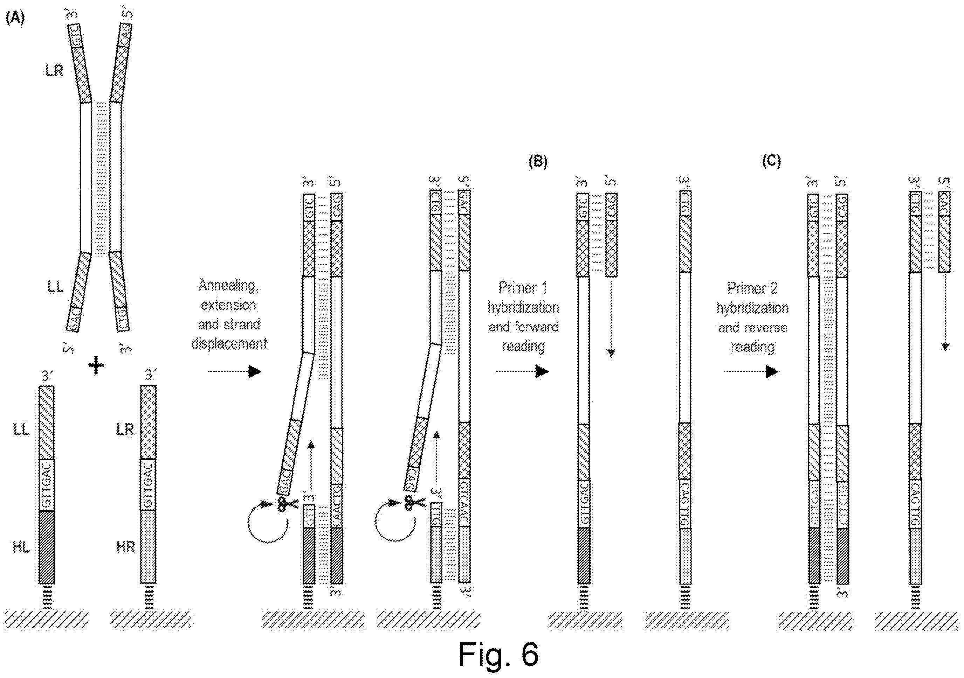

Figure 6: EM-SEq with surface-bound primers can generate amplicons for paired-end next-generation sequencing. (A) Transient annealing between complementary low melting temperature regions LULR present in the template DNA and the surface bound primers allows snap-extension and strand-displacement (as depicted in Figure 3), creating two types of amplification units (as depicted in Figure 4). Figure shows a double stranded template DNA, however other embodiments may use single stranded template DNA as input. (B) After amplification is completed and DNA nicks are filled, complementary DNA strands are dehybridised from surface bound DNA strands and a first sequencing primer is hybridised, allowing forward strand sequencing. (C) After forward reading is completed, second sequencing a second sequencing primer is hybridised, allowing for reverse strand sequencing. Figure shows low melting regions LL and LR being used as sequencing primers, however in other embodiments sequencing primer binding sites may be located anywhere within the boundaries of generated amplicons.
Figure 7 shows a version of solid phase EM-SEq using two temperatures.
Figure 8 shows sixteen different 20 nucleotides-long DNA sequences having either 20%, 30%, 40% or 50% GC content.
Figure 9A shows how the sixteen different sequences of Figure 8 were added in pairs to a 85 nucleotides-long target to serve as flanking motifs (LL and LR) modifying target ends GC content.
Figure 9B shows melting profiles of the created constructs were tested in presence of buffer comprising 70 mM monocationic and 2 mM bicationic salt and a fluorescent double-stranded DNA-binding dye EvaGreen.
Figure 10 shows two full-length amplification template sequences that were designed by attaching respective primer sequences to both ends of an 80 nucleotides-long E. coli ydfU gene fragment. T^ denotes inverted dT blocker moiety.
Figure 11A shows real-time fluorescence data and gel electrophoresis of products from a naive assay run with primers and template design containing 20% GC low-melting point temperature regions.
Figure 11B shows real-time fluorescence data and gel electrophoresis of products from a naive assay run with primers and template design containing 50% GC flanking regions.
Figure 12A shows real-time fluorescence data and gel electrophoresis of products from an optimised assay run with primers and template design containing 20% GC low-melting point temperature regions.
Figure 12B shows real-time fluorescence data and gel electrophoresis of products from an optimised assay run with primers and template design containing 50% GC flanking regions.
Figure 13 shows a full-length template, containing 30 nucleotides-long 66% GC content high melting point regions at the 5' ends (HL and HR, in bold,), recognition sites for Nt.BstNBI nickase, 20 nucleotides-long 20% GC content low melting point regions flanking a target sequence and the sequence of a dead-end product molecule, containing only 20 nucleotides-long 20% GC content low melting point regions flanking a target sequence.
Figure 14 shows real-time fluorescence data that shows the dead-end product can serve as template in the EM-SEq reaction, demonstrating invasion of EM-SEq primers into the transiently single-stranded ends of the molecule.
Figure 15 shows sequences for unmodified forward and reverse primers and hybrid invasion forward and reverse primers.
Figure 16A shows gel electrophoresis of products that shows the reactions containing hybrid invasion primers resulted in pattern of product bands where higher molecular weight products were overrepresented, while lower molecular weight products were underrepresented, compared to the reactions run with unmodified primers.
Figure 16B shows predicted possible product forms.
Figure 17A shows visualisation of EM-SEq reaction products on a glass slide.
Figure 17B shows visualisation of EM-SEq reaction products on the surface of a semiconductor chip.

### DESCRIPTION

One example of Endonuclease-Mediated Shifting Equilibrium Amplification (EM-SEq) is achieved with one pair of primers and input DNA designed according to a particular method (Figure 2).

Prior to amplification, the target DNA sequence is modified such that it is flanked on both ends by adaptor sequences composed of two regions: (i) proximally placed low melting-temperature regions LL and LR (Low-Tm Left and Low-Tm Right) and (ii) truncated endonuclease recognition sites, such as a binding site for a non-palindromic restriction or nicking enzyme (Figure 2A). In some embodiments, there may be no part of the recognition site initially present in the template DNA and its incorporation may occur as the EM-SEq reaction progresses. In other embodiments, a complete recognition site may be already present in the template DNA (Figure 2B). Template DNA may be either double-stranded or single stranded.

EM-SEq uses primers, each composed of three regions: (i) 5'-terminal high melting-temperature region HL or HR (High-Tm Left and High-Tm Right), (ii) centrally placed endonuclease recognition site, matching the truncated recognition site present in the template DNA, and (iii) 3'-terminal low melting-temperature region LL or LR, fully or partially complementary to those present in the template DNA (Figure 2C).

As the ends of the strands to be amplified have been modified with transiently single stranded (breathable) ends, the primers can be hybridised without having to heat denature the sample. Once hybridised, the primer and the adapted 3'- end of the template both undergo extension. Once extended, the ends are no longer breathable as the breathable region is now internal to the sequence.

Low and high melting-temperature regions LL, LR, HL, and HR can be chosen either from sequences found in nature or can be partially or entirely artificial. Instead of modifying the target sequence to contain low melting-temperature regions LL and LR regions, target sequences can be also chosen such that the LL and LR are part of the target sequence found in nature.

The desired difference in melting temperature of the two region types may be achieved in a number of ways. For example, (i) through significantly different GC% content, (ii) use of modified bases that alter thermal stability of the DNA duplex, such as but not limited to locked nucleic acids (LNA), unlocked nucleic acids (UNA) or 8-aza-7-deazaguanosine, and (iii) partial complementary between LL and LR regions present in the primer molecules and those in the template DNA.

At the reaction temperature of thermophilic SDA, which is typically between 50 and 65 degrees Celsius, low-melting temperature regions LL and LR flanking the target sequence allow for transient opening of the DNA duplex ("DNA breathing") at both ends of the template DNA (Figure 3A). In presence of EM-SEq primers, which contain sequences complementary to LL and LR, each of the template DNA ends may exist in two alternative states: annealed with itself or with its matching primer (Figure 3B). Due to a transient and unstable nature of these interactions, the system is governed by a very dynamic kinetic equilibrium, where LL and LR regions continuously bind and dissociate. Kinetics of such binding equilibrium strongly favours the self-annealed state due to the immediate physical proximity of LL and LR regions on the two complementary strands of template DNA.

In presence of a DNA polymerase, however, transient and infrequent annealing of some template molecules with LL and LR regions on matching primers can facilitate "snap-extension" events, where 3'-ends of these template molecules become extended over high melting-temperature regions HL and HR. In contrast to association and dissociation events creating the dynamic binding equilibrium, such extension events are irreversible. Upon 3'-end extension, molecules remain primarily annealed due to being surrounded by high melting temperature region HL or HR on one side and the target sequence on the other.

The snap extension creates a shifting equilibrium effect in accordance with Le Chatelier's principle, whereby any system at equilibrium subjected to a change, e.g. in concentration of its reactants, readjusts itself to counteract the effect of applied change and a new equilibrium is established. Shifting equilibrium effect facilitates conversion of the majority of template molecules into restriction site-regenerated states (Figure 3C). When applied to amplification cycle of SDA, shifting equilibrium effect enabled by differential affinity of low and high melting temperature regions acts on dead-end self-annealed products, regenerating cleaved endonuclease recognition sites (Figure 4). This shifts the balance between products being generated towards molecules that can be amplified exponentially, thus increasing reaction speed and reducing the number of types of amplicon molecules produced down to two major products, both of which contain sequences or moieties present in 5' ends of the primers used.

The transient and infrequent nature of the interaction between primers and the low melting point temperature limits the rate at which the primer is converted to a product containing regenerated endonuclease recognition site and a the full length primer sequence at the 5'-end. The inefficiency of this process results from the fact that the nominal melting point temperature of sequences comprising primer-template duplex is the same as of sequences comprising the duplex formed by self-annealed strands of the complementary terminal low-melting point regions in the double-stranded product (LL and LR). In order to facilitate more efficient hybridization of the primer molecule to the transiently single-stranded low melting point region of the template, the primer sequence complementary to LL or LR regions can be composed of nucleotides with modified bases that stabilise primer-template duplex formation and increase its melting point temperature over the equivalent G/C or A/T base pairs.

In certain embodiments, the extendable 3'- hydroxyl groups on the primer can be generated by strand cleavage of a longer sequence. The longer sequence can have a blocked 3'- end which is non-extendable. The longer sequence contains the full recognition sequence of an endonuclease, and can be cleaved by the endonuclease which cleaves the primer after the released 3- end has been extended (Figure 4b).

Since EM-SEq shifts the balance between generated products towards molecules with regenerated endonuclease recognition sites, as amplification reagents become depleted the reaction plateaus with nicked molecules being predominantly present in the reaction. To avoid generation of nicked amplicon molecules, endonuclease must be inactivated (such as by heat inactivation) before polymerase activity is lost and reagents become completely depleted.

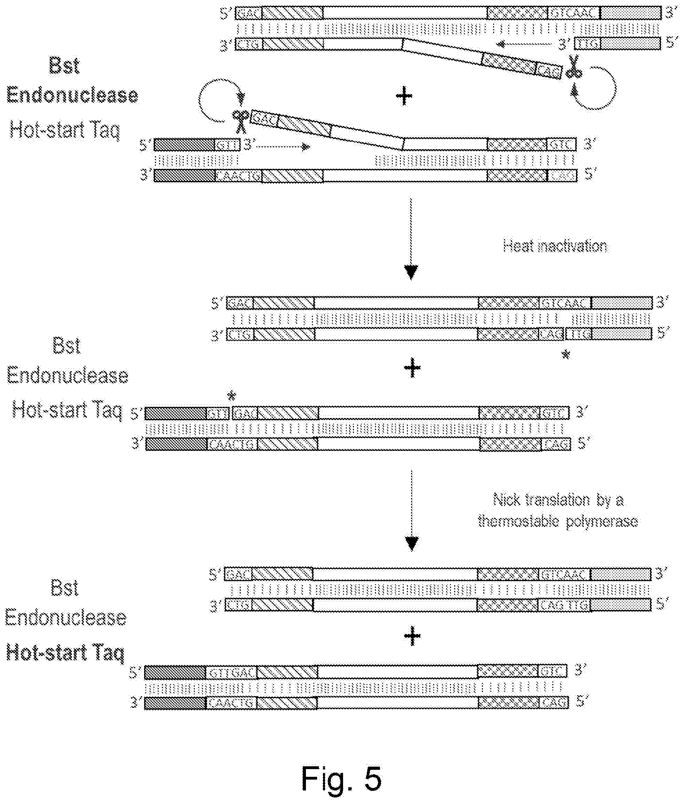

To achieve this, a thermostable strand-displacing DNA polymerase can be used. Alternatively, if a thermosensitive polymerase is used in the reaction, EM-SEq may be prepared with addition of a hot-start thermostable DNA polymerase, without necessity for a strand-displacing activity (e.g. Taq polymerase). In such embodiment, heat inactivation of both the strand-displacing polymerase and the restriction enzyme simultaneously activates the hot-start thermostable polymerase, which subsequently fills the DNA nicks though a process known as "nick translation" (Figure 5).

### EM-SEq in solid-phase amplification for next-generation DNA sequencing devices

When DNA nicks in generated amplicon molecules are filled, most of EM-SEq products are double-stranded and contain the 5'-terminal sequences of one of the two EM-SEq primers used. This enables the use of EM-SEq in generation of surface-bound amplicons when immobilized primers are present in the reaction (Figure 6).

Importantly, in contrast to other isothermal amplification methods, linear production of amplicons through continuous cycles of cleavage and strand displacement does not require annealing of primers on the distal end of the bound amplicon molecules. Because of this, both EM-SEq primers can be immobilized to the surface and there is no need for solution primers to be present in the reaction. This is of particular benefit if the amount of soluble primer is limited due to volume constraints. Also, eliminating primers from solution is expected to vastly reduce off-target amplification happening due to primer dimers.

In the context of next-generation DNA sequencing devices, immobilization of both EM-SEq primers enables solid-phase amplification of both strands of the target sequence at once in the same reaction. The amplification produces an increase in the number of strands in solution, each of which can be captured by an immobilised primer. Following filling of DNA nicks and denaturation of complementary strands, each immobilised primer can be turned into a template strand for sequencing. Using immobilised primers having two different sequences, it becomes possible to perform sequential paired-end reading on both strands (Figure 6).

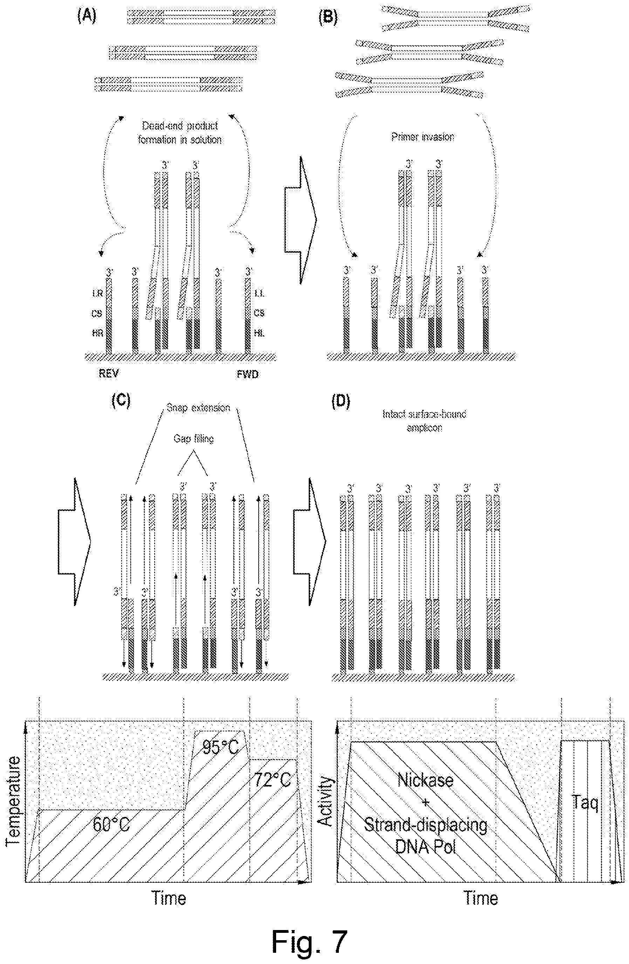

In the EM-SEq reaction described above, binding of primers to low-melting point regions of self-annealed dead-end products and the resulting conversion of these products to extension products with regenerated endonuclease recognition sites and complete primer sequences occurs simultaneously with cleavage and strand extension processes generating further copies of these dead-end products. In order to overcome non-specific interactions between transiently opening low-melting point temperature regions at the reaction temperature, in one EM-SEq embodiment the reaction may be divided into two phases, with cleavage and strand extension processes occurring in a first phase, followed by product conversion occurring in a second phase of the reaction, such that the second phase occurs at a higher temperature than the first phase. In such embodiment, low-melting point temperature regions are design such that they remain largely self-annealed during the first phase, while raising the reaction temperature in the second phase of the reaction enables binding of primers and product conversion. In another EM-SEq embodiment, the two phases may be separated by a nickase inactivation step and gap filling-polymerase activation step such that gap filling and dead-end product conversion both occur in the second phase, driven by the activity of the gap filling-polymerase (Figure 7).

The methods disclosed herein enable the generation of NGS (next generation sequencing) "sequence-ready" DNA fragments. The fragments may represent a whole population from a sample, or may be a targeted subset of the total DNA present in the original template DNA sample. Just those loci of interest are amplified by, for example, polymerase chain reaction, such that the amplicons produced have the template DNA of interest flanked by terminal ends of known sequence. These known end sequences are identical or substantially identical on all the amplicons generated from a given locus, and can be deliberately and controllably asymmetric, with distinct sequences applied to each of the two ends of the amplified fragments. A first known end originates from the 5' region of the first or 'forward' primers and the second known end originates from the 5' region of the second or 'reverse' primers. The amplicons thus produced can be functionally equivalent to adapter-ligated fragments produced in conventional NGS methods, but offer distinct advantages in terms of ease, time and cost of production, as well as quality of the sequencing data subsequently produced. The terminal ends of the amplicons can be amenable to generic 'one-size-fits-all' biochemistry during subsequent manipulations, such as clonal amplification and DNA sequencing, regardless of the locus from which they originate.

The source nucleic acid may be a genomic polynucleotide. The source material may be eukaryotic, prokaryotic, or archaeal. One or more source materials may be provided. The source nucleic acid may represent a fragment of a genome; for example, a single chromosome, or a single genomic locus (for example, for rapid sequencing of allelic polymorphisms). In particular examples the amplification may be specific for pathogenic material within a sample. For example the amplification may select bacterial or viral nucleic acids present within a human sample. Templates may be DNA, RNA, or the cDNA copies thereof.

The methods and primers are agnostic over the subsequent manipulations that generate pools of clonally amplified products (amenable to the generation of clonal populations both on a surface, on a bead or in solution). The technology is also agnostic of the technology that is subsequently used to generate the NGS data, and could be used (for example) with Illumina SBS technology, Ion Torrent or Roche 454 'one base at a time' technologies, or other NGS technologies such as nanopore sequencing. In general, the methods described herein may be advantageous where it is desirable to introduce defined sequences onto the end or ends of specific amplified products.

Also disclosed are kits for performing the method. Disclosed is a kit for the modification of nucleic acid sequences comprising:
a. a first pair of nucleic acid adaptor molecules for modifying the ends of a a population of double stranded nucleic acid sequences, wherein the 5' end of a first strand of the adaptor pair contains part of a recognition site for an endonuclease, the central part of the adaptor pair contains a low melting point region of sequence which, at a temperature of above 37 °C and below 80 °C, is at least transiently single stranded, and the 3' end of the first strand of the adaptor pair and the 5' end of the second strand of the adaptor pair can undergo ligation to both strands of the population of double stranded nucleic acid sequences; and
b. a ligase enzyme.

The kit may include additional components, for example one or more amplification primers or one or more enzymes. The kits may include a strand displacing polymerase. One or more of the reagents may be immobilised, for example the amplification primers can be immobilised.

The amplification can be carried out on a solid support or in the wells of a solid support. Disclosed are arrays having two primer sequences, each primer sequence being composed of three regions: (i) 5'-terminal high melting-temperature region HL or HR (High-Tm Left and High-Tm Right), (ii) centrally placed endonuclease recognition site, matching the truncated recognition site present in the template DNA, and (iii) 3'-terminal low melting-temperature region LL or LR, fully or partially complementary to those present in the template DNA (Figure 2C).

Optionally the immobilised primers can have modified bases in the low melting regions which increase the stability when compared to the native bases. Optionally the primers can have a 3'- block. Optionally the primers can be cleaved at the central endonuclease recognition site prior to extension.

Also disclosed are arrays of amplified polynucleotides made according to the invention. Amplification of the products in discreet wells allows clonal amplification from a single template per well. The wells can be part of a sensor system such as an ISFET sensor to detect proton release. The sensor system can detect pH changes such as for example as seen during nucleotide triphosphate incorporation reactions.

An example of using the method in action may involve the steps of:
Taking a nucleic acid sample. Modifying the sample to include breathable low melting ends as described herein. Taking an array having two primer sequences as disclosed herein, the array being a collection of wells having ISFET sensors. Placing the sample on the array such that the concentration of the molecules in the sample gives rise to an average occupancy of less than one molecule per well. Amplifying the molecules to produce multiple copies of the strands in wells having molecules, where both strands can be amplified. Treating the array to remove any nicks by strand extension. Removing the hybridised strands to make the immobilised strands single stranded. Hybridising a first sequencing primer to a first of the immobilised strands and obtaining a first sequencing read. Removing the first read. Hybridising a second sequencing primer to the second of the immobilised strands and obtaining a second sequencing read, the two reads being from opposite ends of the original double stranded template.

### Examples

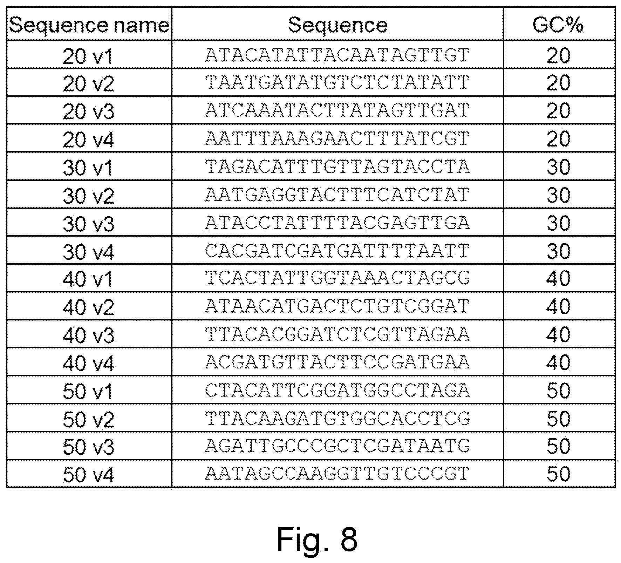

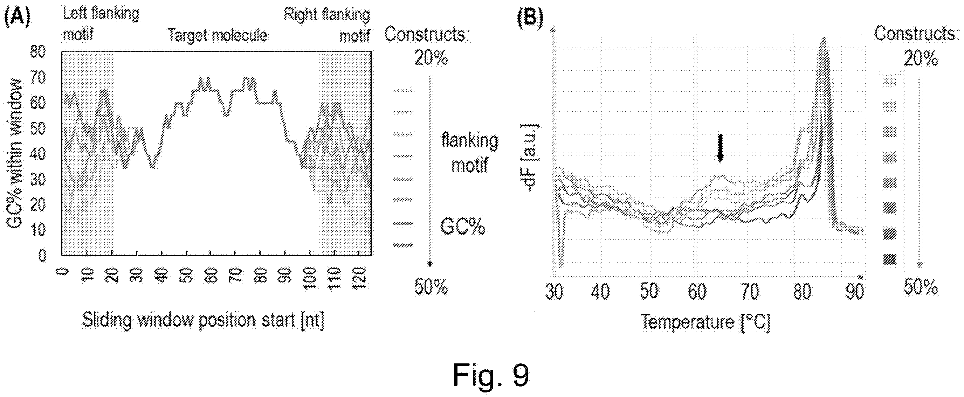

To test the principle of transient opening of target molecule ends, sixteen different 20 nucleotides-long DNA sequences having either 20%, 30%, 40% or 50% GC content were prepared (Figure 8). The sequences were added in pairs to a 85 nucleotides-long target to serve as flanking motifs (LL and LR) modifying target ends GC content (Figure 9A). Melting profiles of the created constructs were tested in presence of buffer comprising 70 mM monocationic and 2 mM bicationic salt and a fluorescent double-stranded DNA-binding dye EvaGreen. Negative differential of recorded fluorescence intensity (-dF) was plotted against incubation temperature in range of 30°C and 90°C for each construct. The resulting curves reveal an increase in -dF between 50°C and 70°C for constructs modified with sequences of low GC% content, indicative of transient opening of construct ends (Figure 9B).

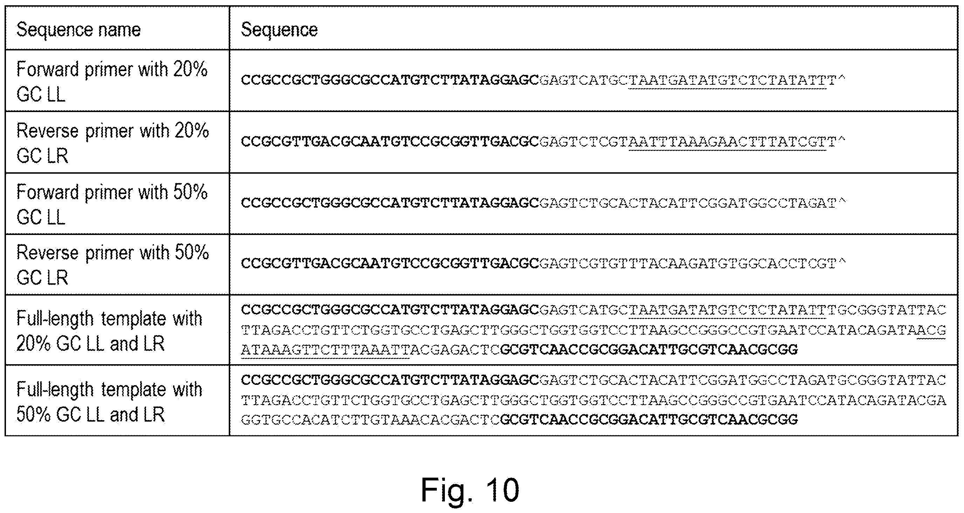

A design of a pair of EM-SEq primers containing a 30 nucleotides-long 66% GC content high melting point region at the 5' end (HL and HR, in bold), a recognition site for Nt.BstNBI nickase and a 20 nucleotides-long 20% GC content low melting point region at the 3' end (LL and LR, underlined). A pair of control primers containing a 20 nucleotides-long 50% GC content region at the 3' end was also designed. In addition, two full-length amplification template sequences were designed by attaching the respective primer sequences to both ends of an 80 nucleotides-long E. coli ydfU gene fragment. T^ denotes inverted dT blocker moiety (Figure 10).

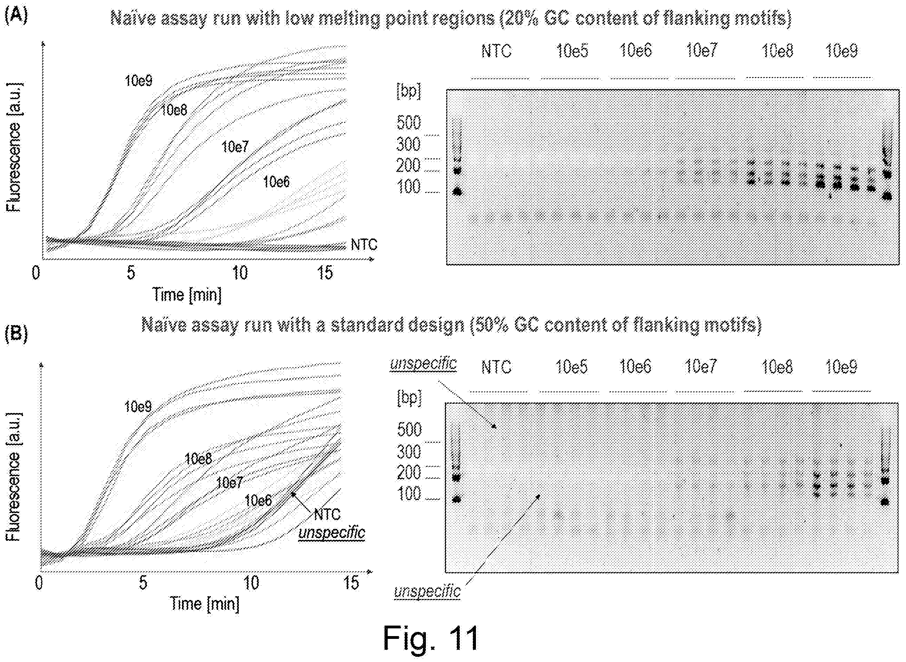

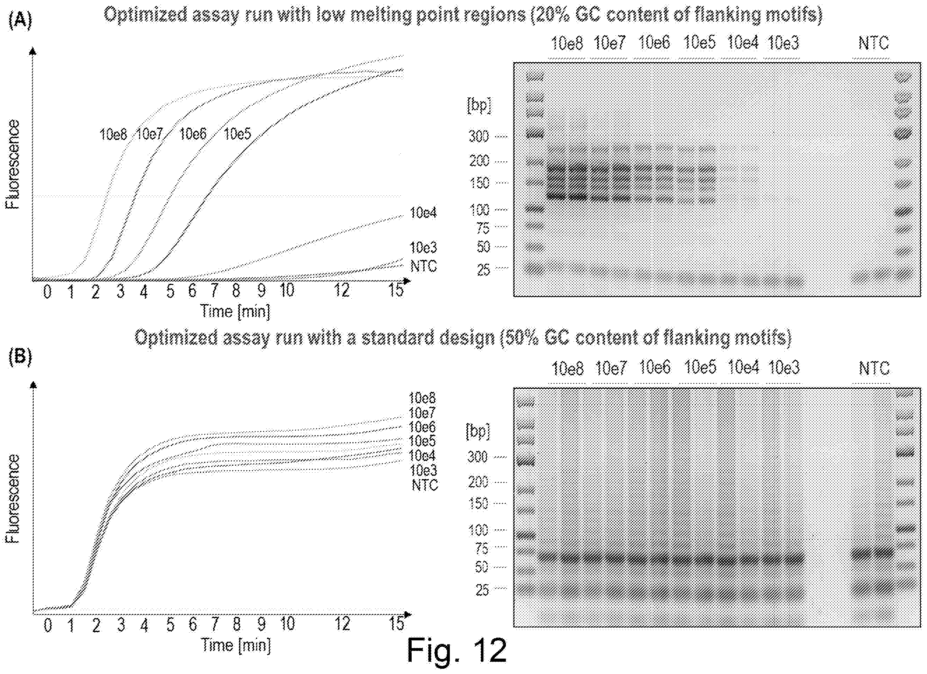

A naive amplification assay was performed in solution with either forward primer with 20% GC LL, reverse primer with 20% GC LR and full-length template with 20% GC LL and LR or forward primer with 50% GC LL, reverse primer with 50% GC LR and full-length template with 50% GC LL and LR in presence of 1X Isothermal Amplification buffer (NEB), 0.32 SYBR Green, 6 mM MgSO₄, 1.4 mM dNTPs, 0.32 U/µl Bst 2.0 WarmStart (NEB) and 0.4 U/µl Nt.BstNBI (NEB). 25 µl reactions containing indicated amount of template copies or no-template control reactions (NTC) were incubated at 60°C for 15 minutes in a qPCR thermocycler. Real-time fluorescence data and gel electrophoresis of products demonstrate that an assay run with primers and template design containing 20% GC low-melting point temperature regions (Figure 11A) showed at least a 10-time higher sensitivity and an improved specificity to the control assay run with primers and template design containing 50% GC flanking regions (Figure 11B). An optimized EM-SEq assay was performed in solution with either forward primer with 20% GC LL, reverse primer with 20% GC LR and full-length template with 20% GC LL and LR or forward primer with 50% GC LL, reverse primer with 50% GC LR and full-length template with 50% GC LL and LR in presence of 1X Isothermal Amplification buffer (NEB), 0.32 SYBR Green, 3 mM MgSO₄, 0.7 mM dNTPs, 35 mM KCI, 5% PEG 8000k, 0.37 U/µl Bst 2.0 WarmStart (NEB) and 0.4 U/µl Nt.BstNBI (NEB). 25 µl reactions containing indicated amount of template copies or no-template control reactions (NTC) were incubated at 60°C for 15 minutes in a qPCR thermocycler. Real-time fluorescence data and gel electrophoresis of products show the assay run with primers and template design containing 20% GC low-melting point temperature regions (Figure 12A) demonstrating a vastly different performance than the control assay run with primers and template design containing 50% GC flanking regions (Figure 12B).

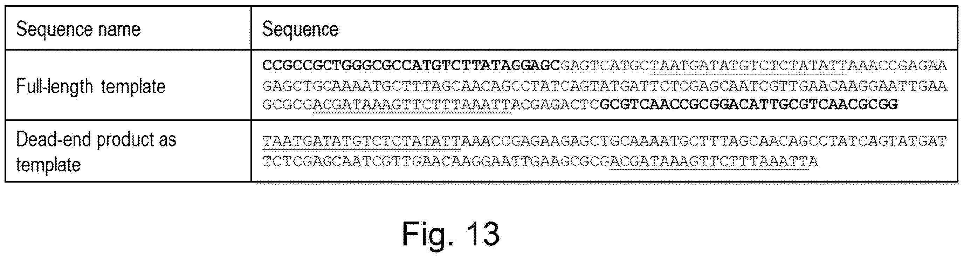

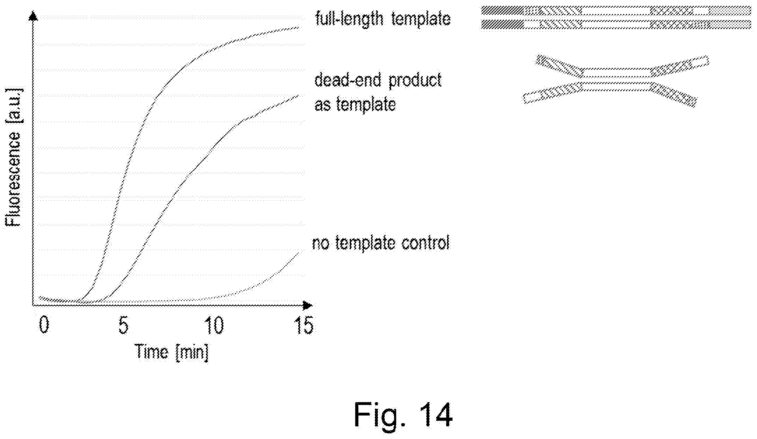

EM-SEq reactions were performed in solution as described above in presence of either a full-length template, containing 30 nucleotides-long 66% GC content high melting point regions at the 5' ends (HL and HR, in bold, Figure 13), recognition sites for Nt.BstNBI nickase, 20 nucleotides-long 20% GC content low melting point regions flanking a target sequence or in presence of a dead-end product molecule as template, containing only 20 nucleotides-long 20% GC content low melting point regions flanking a target sequence. Real-time fluorescence data shows the dead-end product can serve as template in the EM-SEq reaction, demonstrating invasion of EM-SEq primers into the transiently single-stranded ends of the molecule (Figure 14).

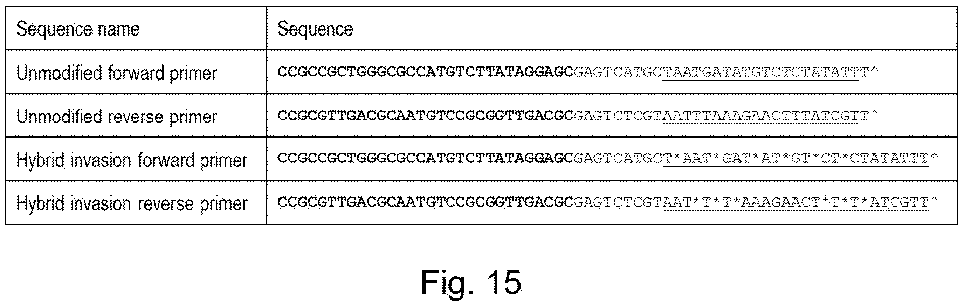

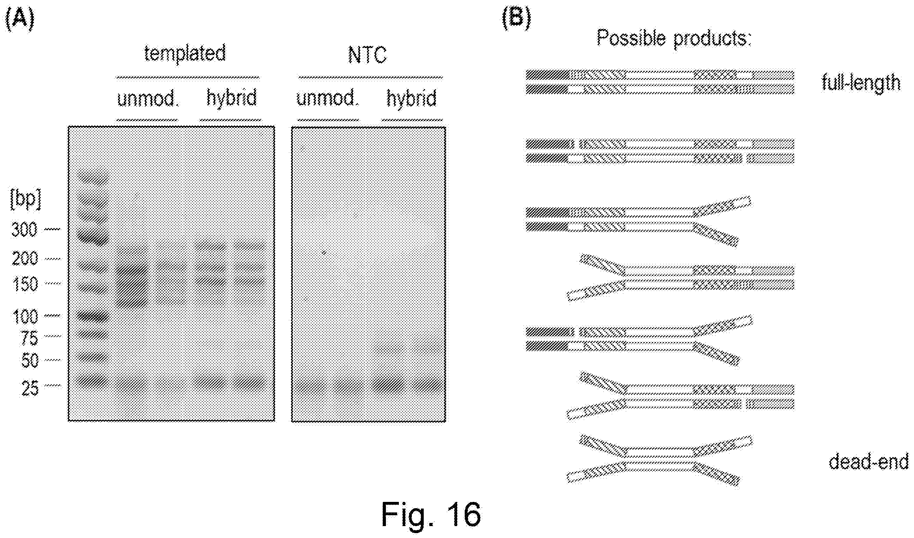

EM-SEq reactions were performed in solution as described above in presence of either unmodified EM-SEq primers comprising a 30 nucleotides-long 66% GC content high melting point region at the 5' end (HL and HR, in bold, Figure 15), a recognition site for Nt.BstNBI nickase and a 20 nucleotides-long 20% GC content low melting point region at the 3' end (LL and LR, underlined) or hybrid invasion primers where six of the thymidine bases in the 20% GC content low melting point regions were replaced with SuperT bases (5-hydroxybutynl-2'-deoxyuridine, denoted T*). Gel electrophoresis of products shows the reactions containing hybrid invasion primers resulted in pattern of product bands where higher molecular weight products were overrepresented, while lower molecular weight products were underrepresented, compared to the reactions run with unmodified primers (Figure 16A). This indicates the hybrid invasion EM-SEq reactions promoted production of molecules with regenerated ends. Figure 16B lists the predicted product forms.

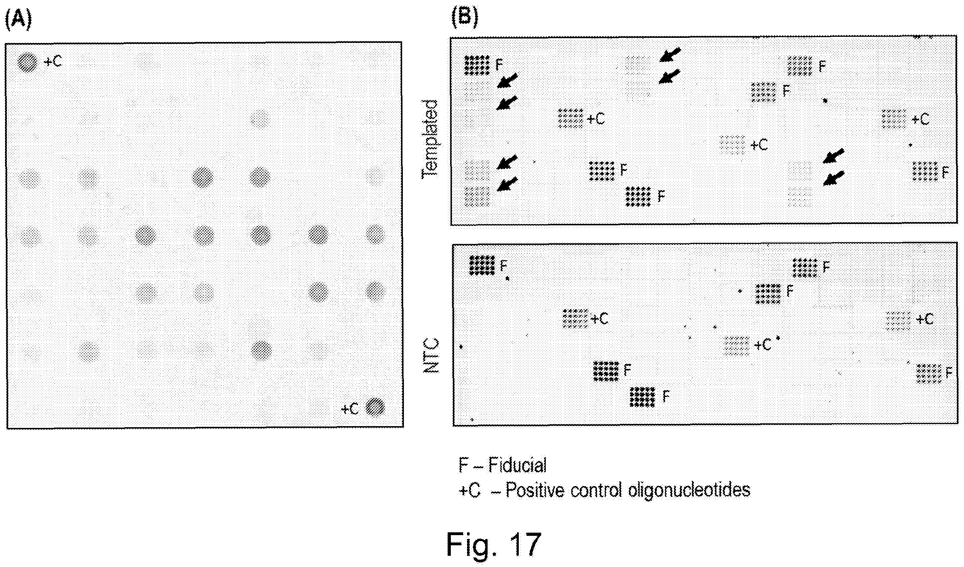

EM-SEq reactions were performed as described above on a glass slide in presence of two EM-SEq primers spotted on the surface in a 1:1 ratio (Figure 17A). Spots containing a mix of the two EM-SEq primers vary in different designs of the linker moieties needed for oligonucleotide immobilisation onto the surface. After the reaction, slides were washed, complementary DNA strands were dehybridized with NaOH and amplified DNA was visualised through hybridisation of a fluorescent probe specific to the amplified target.

EM-SEq reactions were also performed as described above on a surface of a semiconductor chip capable of supporting DNA sequencing though detection of proton release (Figure 17B). Arrows indicate 4x5 spot arrays were EM-SEq primers were immobilized. All areas other than EM-SEq primers, positive control oligonucleotides or fiducial contain unrelated primers. After the reaction, slides were washed, complementary DNA strands were dehybridized with NaOH and amplified DNA was visualised through hybridisation of a fluorescent probe specific to the amplified target.

## Claims

1. A method for the strand displacement amplification of a population of double stranded nucleic acid sequences comprising:
a. modifying the ends of the strands in the population such that at least one of the ends contains a low melting point region of sequence which, at a temperature of 37-80 °C, is at least transiently single stranded;
b. copying the population of nucleic acid molecules having low melting point ends using one or more amplification primers which hybridise to the low melting point ends, wherein the primers have a 5' single stranded section beyond the 3' end of the template population of nucleic acid molecules such that the 3' end of the template is extended to form a complete recognition site for an endonuclease, and the 3' end of the primer is extended by strand displacement to copy the template;
c. using the complete recognition site for the endonuclease to nick the extended strand, thereby releasing a free 3'-OH group within the primer; and
d. extending the freed 3'-OH group by strand displacement to re-copy the template,
wherein steps b, c and d are performed isothermally, thereby resulting in the strand displacement amplification of the population of double stranded nucleic acid sequences.

2. The method according to claim 1, step a. wherein the ends of strands in the population are modified such that at least one of the ends contains a low melting region of sequence which, at a temperature of 37-65°C, is at least transiently single stranded.

3. The method according to claim 1 or claim 2 wherein the amplification is carried out with a single amplification primer, thereby copying one strand of the population of double stranded nucleic acid sequences or the amplification is carried out with two amplification primers, thereby copying both strands of the population of double stranded nucleic acid sequences.

4. The method of claim 3 wherein one or more of the amplification primers are immobilised on a solid support.

5. The method of any one of claims 1 to 4 wherein extension steps b and d are performed using a strand displacing polymerase.

6. The method according to any one of claims 1 to 5 wherein the ends of the strands in the population are modified by adaptor ligation.

7. The method according to any one of claims 1 to 6 wherein the modified ends of the strands in the population are obtained using extension of a primer having said modification.

8. The method according to any one of claims 1 to 7 wherein the modified ends contain part of a recognition site for an endonuclease.

9. The method according to any one of claims 1 to 8 wherein the sequence of the amplification primer having a 5' single stranded section beyond the 3' end of the template population has a complete strand of a recognition site for an endonuclease in said single stranded section, and the complete double stranded recognition site is made by strand extension.

10. The method according to any one of claims 1 to 9 wherein the low melting point region contains a mis-matched base pair or a region of 20 nucleotides having less than 30% GC content.

11. The method according to any one of claims 1 to 10 wherein the isothermal amplification temperature is 50-65 °C.

12. The method according to any one of claims 1 to 11 wherein the nick in the extended strand is generated using a nicking endonuclease selected from Nt.BspQl, Nt.CviPII, Nt.BstNBI, Nb.BsrDI, Nb.BtsI, Nt.Alwl, Nb.BbvCl, Nt.BbvCl or Nb.Bsml.

13. The method according to any one of claims 1 to 12 wherein the extension is performed using a non-natural dNTP which generates a non cleavable nucleic acid backbone.

14. The method according to any one of claims 1 to 13 wherein the primers are blocked at their 3' end by a blocking moiety, preventing 3' end extension before nicking occurs.

15. A kit for the modification of nucleic acid sequences comprising:
a. a first pair of nucleic acid adaptor molecules for modifying the ends of a a population of double stranded nucleic acid sequences, wherein the 5' end of a first strand of the adaptor pair contains part of a recognition site for an endonuclease, the central part of the adaptor pair contains a low melting point region of sequence which, at a temperature of 37-80 °C, is at least transiently single stranded, and the 3' end of the first strand of the adaptor pair and the 5' end of the second strand of the adaptor pair can undergo ligation to both strands of the population of double stranded nucleic acid sequences; and
b. a ligase enzyme.

## Patentansprüche

1. Verfahren zur Strangverdrängungsamplifikation einer Population von doppelsträngigen Nukleinsäuresequenzen, umfassend:
a. Modifizieren der Enden der Stränge in der Population, sodass mindestens eines der Enden eine Sequenzregion niedrigen Schmelzpunkts enthält, der bei einer Temperatur von 37-80 °C zumindest vorübergehend einzelsträngig ist,
b. Kopieren der Population von Nukleinsäuremolekülen, die Enden niedrigen Schmelzpunkts aufweisen, unter Verwendung eines oder mehrerer Amplifikationsprimer, die mit den Enden niedrigen Schmelzpunkts hybridisieren, wobei die Primer einen 5'-Einzelstrangabschnitt jenseits des 3'-Endes der Templatepopulation von Nukleinsäuremolekülen aufweisen, sodass das 3'-Ende des Templates verlängert wird, um eine vollständige Erkennungsstelle für eine Endonuklease zu bilden, und das 3'-Ende des Primers durch Strangverdrängung verlängert wird, um das Template zu kopieren,
c. Verwenden der vollständigen Erkennungsstelle für die Endonuklease, um den verlängerten Strang zu brechen, wodurch eine freie 3'-OH-Gruppe innerhalb des Primers freigesetzt wird, und
d. Verlängern der befreiten 3'-OH-Gruppe durch Strangverdrängung, um das Template erneut zu kopieren,
wobei die Schritte b, c und d isotherm ausgeführt werden, was in der Strangverdrängungsamplifikation der Population doppelsträngiger Nukleinsäuresequenzen resultiert.

2. Verfahren nach Anspruch 1, Schritt a., wobei die Strangenenden in der Population modifiziert sind, sodass mindestens eines der Enden eine niedrig schmelzende Sequenzregion enthält, die bei einer Temperatur von 37-65 °C zumindest vorübergehend einzelsträngig ist.

3. Verfahren nach Anspruch 1 oder Anspruch 2, wobei die Amplifikation mit einem einzigen Amplifikationsprimer durchgeführt wird, wodurch ein Strang der Population doppelsträngiger Nukleinsäuresequenzen kopiert wird, oder die Amplifikation mit zwei Amplifikationsprimern durchgeführt wird, wodurch beide Stränge der Population doppelsträngiger Nukleinsäuresequenzen kopiert werden.

4. Verfahren nach Anspruch 3, wobei einer oder mehrere der Amplifikationsprimer auf einem festen Träger immobilisiert sind.

5. Verfahren nach einem der Ansprüche 1 bis 4, wobei die Verlängerungsschritte b und d unter Verwendung einer strangverdrängenden Polymerase ausgeführt werden.

6. Verfahren nach einem der Ansprüche 1 bis 5, wobei die Enden der Stränge in der Population durch Adaptor-Ligation modifiziert werden.

7. Verfahren nach einem der Ansprüche 1 bis 6, wobei die modifizierten Enden der Stränge in der Population unter Verwendung einer Verlängerung eines Primers erlangt werden, der die Modifikation aufweist.

8. Verfahren nach einem der Ansprüche 1 bis 7, wobei die modifizierten Enden einen Teil einer Erkennungsstelle für eine Endonuklease enthalten.

9. Verfahren nach einem der Ansprüche 1 bis 8, wobei die Sequenz des Amplifikationsprimers, der einen einzelsträngigen 5'-Abschnitt jenseits des 3'-Endes der Templatepopulation aufweist, einen vollständigen Strang einer Erkennungsstelle für eine Endonuklease in diesem einzelsträngigen Abschnitt aufweist und die vollständige doppelsträngige Erkennungsstelle durch Strangverlängerung gefertigt wird.

10. Verfahren nach einem der Ansprüche 1 bis 9, wobei die Region niedrigen Schmelzpunkts ein fehlgepaartes Basenpaar oder eine Region von 20 Nukleotiden, das einen Gehalt von weniger als 30 % GC aufweist, enthält.

11. Verfahren nach einem der Ansprüche 1 bis 10, wobei die isotherme Amplifikationstemperatur 50-65 °C ist.

12. Verfahren nach einem der Ansprüche 1 bis 11, wobei der Bruch in dem verlängerten Strang unter Verwendung einer Bruch-Endonuklease erzeugt wird, ausgewählt aus Nt.BspQI, Nt.CviPII, Nt.BstNBI, Nb.BsrDI, Nb.BtsI, Nt.AlwI, Nb.BbvCI, Nt.BbvCI oder Nb.Bsml.

13. Verfahren nach einem der Ansprüche 1 bis 12, wobei die Verlängerung unter Verwendung eines nicht-natürlichen dNTPs durchgeführt wird, das ein nicht-spaltbares Nukleinsäuregerüst erzeugt.

14. Verfahren nach einem der Ansprüche 1 bis 13, wobei die Primer an ihrem 3'-Ende durch eine blockierende Einheit blockiert sind, die eine Verlängerung des 3 '-Endes verhindert, bevor ein Bruch erfolgt.

15. Kit zur Modifikation von Nukleinsäuresequenzen, umfassend:
a. ein erstes Paar von Nukleinsäure-Adaptormolekülen zum Modifizieren der Enden einer Population von doppelsträngigen Nukleinsäuresequenzen, wobei das 5'-Ende eines ersten Strangs des Adaptorpaars einen Teil einer Erkennungsstelle für eine Endonuklease enthält, der mittlere Teil des Adaptorpaars eine Sequenzregion niedrigen Schmelzpunkts enthält, die bei einer Temperatur von 37-80 °C zumindest vorübergehend einzelsträngig ist, und das 3'-Ende des ersten Strangs des Adaptorpaars und das 5'-Ende des zweiten Strangs des Adaptorpaars eine Ligation an beide Stränge der Population doppelsträngiger Nukleinsäuresequenzen eingehen können; und
b. ein Ligase-Enzym.

## Revendications

1. Procédé d'amplification par déplacement de brin d'une population de séquences d'acides nucléiques double brin comprenant :
a. la modification des extrémités des brins dans la population de sorte qu'au moins l'une des extrémités contienne une région de séquence à bas point de fusion qui, à une température de 37-80° C, est au moins transitoirement à simple brin ;
b. la copie de la population de molécules d'acides nucléiques ayant des extrémités à bas point de fusion en utilisant une ou plusieurs amorces d'amplification qui s'hybrident aux extrémités à bas point de fusion, dans lequel les amorces ont une section à simple brin 5' au-delà de l'extrémité 3' de la population matrice de molécules d'acides nucléiques de sorte que l'extrémité 3' de la matrice soit étendue pour former un site de reconnaissance complet pour une endonucléase, et l'extrémité 3' de l'amorce soit étendue par déplacement de brin pour copier la matrice ;
c. l'utilisation du site de reconnaissance complet pour l'endonucléase pour couper le brin étendu, libérant ainsi un groupe 3'-OH libre dans l'amorce ; et
d. l'extension du groupe 3'-OH libéré par déplacement de brin pour recopier la matrice,
dans lequel les étapes b, c et d sont réalisées de manière isotherme, entraînant ainsi l'amplification par déplacement de brin de la population de séquences d'acides nucléiques double brin.

2. Procédé selon la revendication 1, étape a. dans lequel les extrémités des brins dans la population sont modifiées de sorte qu'au moins une des extrémités contienne une région de séquence à bas point de fusion qui, à une température de 37-65° C, est au moins transitoirement à simple brin.

3. Procédé selon la revendication 1 ou la revendication 2, dans lequel l'amplification est réalisée avec une seule amorce d'amplification, copiant ainsi un brin de la population de séquences d'acides nucléiques double brin ou l'amplification est réalisée avec deux amorces d'amplification, copiant ainsi les deux brins de la population de séquences d'acides nucléiques double brin.

4. Procédé selon la revendication 3, dans lequel une ou plusieurs des amorces d'amplification sont immobilisées sur un support solide.

5. Procédé selon l'une quelconque des revendications 1 à 4, dans lequel les étapes d'extension b et d sont réalisées en utilisant une polymérase à déplacement de brin.

6. Procédé selon l'une quelconque des revendications 1 à 5, dans lequel les extrémités des brins dans la population sont modifiées par ligature d'adaptateur.

7. Procédé selon l'une quelconque des revendications 1 à 6, dans lequel les extrémités modifiées des brins dans la population sont obtenues en utilisant l'extension d'une amorce ayant ladite modification.

8. Procédé selon l'une quelconque des revendications 1 à 7, dans lequel les extrémités modifiées contiennent une partie d'un site de reconnaissance pour une endonucléase.

9. Procédé selon l'une quelconque des revendications 1 à 8, dans lequel la séquence de l'amorce d'amplification ayant une section simple brin 5' au-delà de l'extrémité 3' de la population de matrice a un brin complet d'un site de reconnaissance pour une endonucléase dans ladite section simple brin, et le site de reconnaissance double brin complet est réalisé par extension de brin.

10. Procédé selon l'une quelconque des revendications 1 à 9, dans lequel la région à bas point de fusion contient une paire de base mésappariée ou une région de 20 nucléotides ayant une teneur en GC inférieure à 30 %.

11. Procédé selon l'une quelconque des revendications 1 à 10, dans lequel la température d'amplification isotherme est de 50-65° C.

12. Procédé selon l'une quelconque des revendications 1 à 11, dans lequel l'entaille dans le brin étendu est générée en utilisant une endonucléase par entaille choisie parmi Nt.BspQI, Nt.CviPII, Nt.BstNBI, Nb.BsrDI, Nb.BtsI, Nt.AlwI, Nb.BbvCI, Nt.BbvCI ou Nb.Bsml.

13. Procédé selon l'une quelconque des revendications 1 à 12, dans lequel l'extension est réalisée en utilisant un dNTP non naturel qui génère un squelette d'acides nucléiques non clivable.

14. Procédé selon l'une quelconque des revendications 1 à 13, dans lequel les amorces sont bloquées à leur extrémité 3' par une fraction de blocage, empêchant l'extension de l'extrémité 3' avant que l'entaille ne se produise.

15. Kit pour la modification de séquences d'acides nucléiques comprenant :
a. une première paire de molécules d'acides nucléiquesadaptateurs pour modifier les extrémités d'une population de séquences d'acides nucléiques double brin, dans laquelle l'extrémité 5' d'un premier brin de la paire d'adaptateurs contient une partie d'un site de reconnaissance pour une endonucléase, la partie centrale de la paire d'adaptateurs contient une région de séquence à bas point de fusion qui, à une température de 37-80° C, est au moins transitoirement à brin unique, et l'extrémité 3' du premier brin de la paire d'adaptateurs et l'extrémité 5' du second le brin de la paire d'adaptateurs peut subir une ligature aux deux brins de la population de séquences d'acides nucléiques double brin ; et
b. une enzyme ligase.
